# EUROPEAN PATENT APPLICATION

(11) **EP 4 079 296 A1**
(43) Date of publication of application: **26.10.2022**
(21) Application number: 22164295.2
(22) Date of filing: 25.03.2022
(51) Int. Cl.: A61K 9/20, A61K 9/24

(54) **A BILAYER TABLET FORMULATION COMPRISING AMORPHOUS DAPAGLIFLOZIN AND METFORMIN**

(30) Priority: 21.04.2021 TR 202106927
(71) Applicant: SANOVEL ILAC SANAYI VE TICARET A.S., Istanbul 34460 (TR)
(72) Inventor: GULER, Tolga, Istanbul (TR); PEHLIVAN AKALIN, Nur, Istanbul (TR); SUNEL, Fatih, Istanbul (TR); SAHPAZ CERIT, Filiz, Istanbul (TR); AVERBEK, Seda, Istanbul (TR); MUTLU, Onur, Istanbul (TR)
(74) Representative: Sevinç, Erkan

(57) **Abstract**

The invention relates to a bilayer tablet comprising metformin in the form of the free base or in the form of pharmaceutically acceptable salts, crystalline polymorph thereof and amorphous dapagliflozin, and at least one pharmaceutically acceptable excipient, wherein the tablet is free of magnesium stearate. Also, the combination has an immediate release layer and an extended release layer.

## Description

### Field of Invention

The invention relates to a bilayer tablet comprising metformin in the form of the free base or in the form of pharmaceutically acceptable salts, crystalline polymorph thereof and amorphous dapagliflozin, and at least one pharmaceutically acceptable excipient, wherein the tablet is free of magnesium stearate. Also, the combination has immediate release layer and an extended release layer.

### Background of the Invention

Diabetes mellitus, known as diabetes, is a lifelong chronic disease resulting from the pancreas's inability to produce enough insulin or the body's ability to use insulin effectively, and it continues with the reduction of insulin-producing cells. Blood glucose rises with absent or non-functioning insulin hormone. The resulting high blood sugar causes classical symptoms such as polyuria (frequent urination), polydipsia (increased thirst) and polyphagia (increased hunger). Currently, there are three types of diabetes: type 1 diabetes (type 1), type 2 diabetes (type 2) and gestational diabetes. Gestational diabetes is seen in pregnant women who develop high blood sugar levels. Type 1 diabetes is caused by the body's inability to produce insulin, requiring insulin injection. Finally, with type 2 diabetes, the body either resists the effects of insulin or does not produce enough insulin to maintain a normal glucose level. Of these three types of diabetes, type 2 diabetes is the most common type, affecting more than 171 million people worldwide.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

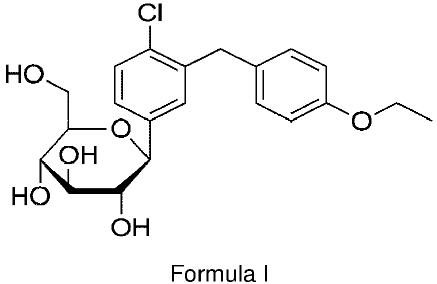

Dapagliflozin was first disclosed in patent US 6515117 (2003, Bristol-Myers Squibb).

Metformin is an antihyperglycemic agent that improves glucose tolerance and lowers both basal and postprandial plasma glucose levels by different mechanisms than other oral antidiabetic agents. Metformin decreases hepatic gluconeogenesis, decreases intestinal absorption of glucose, and improves insulin sensitivity by increasing peripheral glucose uptake and utilization. With metformin therapy, insulin secretion remains unchanged while fasting insulin levels and day-long plasma insulin response may actually decrease. Metformin, also known as N, N-dimethylimidodicarbonimidic diamide or 1,1-Dimethylbiguanide or N, N-dimethyldiguanide or N, N-Dimethylguanylguanidine, is represented by the chemical structure in Formula II.

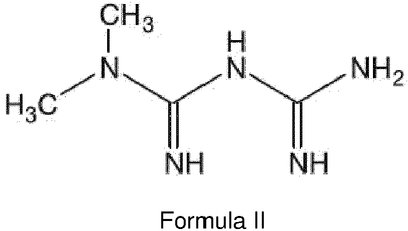

Metformin was first disclosed in patent US 3174901 (1965, Jan Marcel Didier Aron-Samuel).

EP2498758B1 discloses bilayer tablets comprising a first layer, wherein the first layer is extended release metformin and a second layer, wherein the second layer is immediate release dapagliflozin molecule.

EP2498759B1 discloses a process for the preparation of an immediate release formulation comprising a combination of dapagliflozin and metformin.

WO 2017/098481 discloses an effervescent combination comprising metformin with retained carbon dioxide content of at least 90% of the input blend and a different antidiabetic agent.

Unexamined patent TR2012/02948 discloses a tablet form comprising a combination of metformin hydrochloride and dapagliflozin and a core tablet coated with a coating solution containing extended release metformin and dapagliflozin.

The main advantage of bilayer tablet formulations comprising dapagliflozin or a pharmaceutically acceptable salt thereof with metformin or a pharmaceutically acceptable salt thereof compared to other drug forms is the immediate effect of the drug experienced by the target group of patients, which continues throughout the day. Thus, the target patient group does not need to use drugs repeatedly.

In the state of the art, there are tablet configurations with layers that provide immediate and extended releases. Immediate release layers balance the amount of glucose in the blood immediately, while the extended release layers maintain the blood sugar balance over an extended period. But these configurations have undesired properties. Following administration, disintegration and dissolution begin in the immediate and extended release layers and the active substance diffuses into the environment. However, if the immediate release layer does not diffuse fast enough, the polymers that release and control the release in extended release layers creates a diffusion barrier surrounding the tablet. This barrier prevents the immediate release layer, which does not release fast enough, from diffusing and the drug from reaching its desired efficiency. As a result, the desired release profile cannot be achieved. The present formulations require additional time for the onset of action. This is undesirable for the patient. The early onset of the treatment process directly affects the patient's quality of life.

In addition, the solubility of the metformin molecule is high whereas the solubility of the dapagliflozin molecule is low. Achieving the intended release profile is quite difficult. Also, dapagliflozin is hygroscopic in nature. It absorbs moisture and forms sticky lumps which are difficult to process and handle, and which may ultimately lead to content uniformity issues in the dosage form.

The selection and proportion of excipients with appropriate release profile for the desired drug configuration, promoting the release from the immediate release layer at the desired rate is of critical importance.

In order to eliminate the mentioned problems, it is obvious that an innovation is needed in the art that will facilitate production of daily single-dose formulations with appropriate release profile comprising metformin and amorphous dapagliflozin.

In this invention, to overcome these problems mentioned above, a bilayer tablet comprising metformin in the form of the free base or in the form of pharmaceutically acceptable salts, crystalline polymorph thereof and amorphous dapagliflozin is provided without magnesium stearate. Also, the bilayer tablet has been developed by using standard techniques which is simple and cost-effective method.

### Background and Description of the Invention

The main object of the present invention is to provide a bilayer tablet with high stability, having desired level of dissolution rate and compressibility which overcomes the above-described problems of active agents in prior art and have additive advantages over them.

The object of the present invention is to provide an improved bilayer tablet formulation comprising metformin in the form of the free base or in the form of pharmaceutically acceptable salts, crystalline polymorph thereof and amorphous dapagliflozin to be used in the treatment of type 2 diabetes, using suitable excipients.

The object of the present invention is to combine metformin in the form of the free base or in the form of pharmaceutically acceptable salts, crystalline polymorph thereof and amorphous dapagliflozin in a single dosage form while maintaining stability and improving the dissolution profile thanks to the said bilayer form which is free of magnesium stearate.

According to an embodiment of the present invention, a pharmaceutical bilayer tablet comprises;
a. first layer comprising metformin in the form of the free base or in the form of pharmaceutically acceptable salts, crystalline polymorph thereof and at least one pharmaceutically acceptable excipient,
b. second layer comprising amorphous dapagliflozin, and at least one pharmaceutically acceptable excipient,
wherein the tablet is free of magnesium stearate.

Magnesium stearate serves both as glidant and lubricant in solid oral pharmaceutical formulations. Although providing high flowability in formulations, in the present invention, we found that magnesium stearate has high moisture retention and increases destabilization rate by increasing degradation. This excipient has caused problems due to the characteristic of the active agents in layers comprising both dapagliflozin and metformin, e.g. stability and compressibility. Therefore, magnesium stearate was not used in these bilayer tablets.

According to an embodiment of the present invention, the first layer has extended release formulation and the second layer has immediately release formulation.

According to an embodiment of the present invention, at least one pharmaceutically acceptable excipient is selected from fillers, binders, disintegrants, matrix agents, glidants/lubricants or mixtures thereof.

Suitable fillers are selected from the group comprising microcrystalline cellulose (PH 302), anhydrous lactose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to an embodiment of the present invention, using filler ensures homogeneous distribution of dapagliflozin, which is used in small amounts in the formulation. The filler is microcrystalline cellulose or anhydrous lactose.

According to an embodiment of the present invention, the amount of fillers is 5.0% to 30.0% by weight. This ratio provides the desired dissolution profile and content uniformity.

Suitable binders are selected from the group comprising hydroxypropyl methylcellulose, carboxymethylcellulose sodium, polyvinylpyrrolidone (povidone), lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

According to an embodiment of the present invention, the binder is hydroxypropyl methylcellulose (K100 LV) or carboxymethylcellulose sodium.

According to an embodiment of the present invention, the amount of binders is 1.0% to 15.0% by weight in the total composition. Preferably, the amount of binders is 3.0% to 10.0% by weight in the total composition. This provides the desired dissolution profile.

Suitable disintegrants are selected from the group comprising croscarmellose sodium, crospovidone, sodium starch glycollate, sodium alginate, gums, starch and magnesium aluminum silicate or a mixture thereof.

It has been surprisingly found that, according to an embodiment of the present invention, the weight ratio of disintegrant to amorphous dapagliflozin is 1.0 - 4.0, preferably 1.2 - 3.5 in the immediate release layer provides bilayer tablet formulation of dapagliflozin with good dissolution and solubility, thus high bioavailability.

According to an embodiment of the present invention, the disintegrant is croscarmellose sodium.

According to an embodiment of the present invention, the amount of disintegrants is 0.1% to 5.0% by weight in the total composition. This amount of disintegrants provides the desired dissolution profile.

Suitable glidants/lubricants are selected from group comprising colloidal silicon dioxide, sodium stearyl fumarate, magnesium oxide, starch, silica colloidal anhydrous, talc, polyethylene glycol, stearic acid, aluminum silicate, magnesium silicate, colloidal silica or mixtures thereof.

According to an embodiment of the present invention, the glidants/lubricants is colloidal silicon dioxide or sodium stearyl fumarate or mixtures thereof.

According to an embodiment of the present invention, the amount of glidants/lubricants is 0.01% to 6.0% by weight in the total composition.

According to an embodiment of the present invention, the extended release formulation in first layer is prepared using matrix agents.

Suitable matrix agents are selected from the group comprising hydroxypropylmethyl cellulose, ethyl cellulose, polymethylmethacrylate derivatives's, poloxamer, polyvinyl alcohol, xanthan gum, sodium alginate, polymethacrylates, polyacrylamide, hydroxypropyl cellulose, polyvinyl acetate, cellulose acetate phthalate, ethylene vinyl acetate, methyl aminoethyl methacrylate, neutral methacrylic acid esters, polylactide, polylactide co-glycolide, diethyl aminoethyl methacrylate or mixtures thereof.

According to an embodiment of the present invention, the matrix agent is hydroxypropyl methyl cellulose (K100 MCR/ K100M/ CN10T).

According to an embodiment of the present invention, the amount of matrix agents is 10.0% to 25.0% by weight in the total composition.

According to an embodiment of the present invention, the bilayer tablet is coated with at least one film coating agent.

Suitable film coating agents are selected from the group comprising polymethacrylates, hydroxypropyl methylcellulose, lactose monohydrate, talc, hydroxypropyl cellulose, polyvinyl alcohol (PVA), polyethylene glycol (PEG), glycerin, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to an embodiment of the present invention, the film coating agents are selected from the group comprising polyvinyl alcohol, talc, titanium dioxide, polyethylene glycol, yellow iron oxide, iron oxide yellow, black iron oxide.

According to an embodiment of the present invention, the amount of film coating agents is 1.0% to 5.0% by weight in the total composition.

In this invention, the combination comprising amorphous dapagliflozin and metformin in the form of the free base or in the form of pharmaceutically acceptable salts, crystalline polymorph thereof is stable. The combination does not show incompatibilities, degradation problems, or extraction problems with certain excipients such as amorphous dapagliflozin, metformin in the form of the free base or in the form of pharmaceutically acceptable salts, crystalline polymorph thereof, microcrystalline cellulose, lactose, croscarmellose sodium, carboxymethylcellulose sodium (7 HF), hydroxypropylmethylcellulose (K100 LV), hydroxypropylmethylcellulose (K100 MCR/ K100M/ CN10T), colloidal silicon dioxide, sodium stearyl fumarate and coating agents.

The present invention provides bilayer tablets comprising an extended release formulation, wherein the first layer comprises metformin in the form of the free base or in the form of pharmaceutically acceptable salts, crystalline polymorph thereof as active ingredient, hydroxypropylmethylcellulose (K100 LV) or carboxymethylcellulose sodium (7 HF) as binder, hydroxypropylmethylcellulose (K100 MCR/ K100M/ CN10T) as a matrix agent, sodium stearyl fumarate as glidant/lubricant, as well as an immediate release formulation of amorphous dapagliflozin wherein the second layer comprises amorphous dapagliflozin as active agent, microcrystalline cellulose and anhydrous lactose as a filler, croscarmellose sodium as disintegrant, colloidal silicon dioxide and sodium stearyl fumarate as glidant/ lubricant.

According to an embodiment of the present invention, the first layer comprises metformin in the form of the free base or in the form of pharmaceutically acceptable salts, crystalline polymorph thereof as active ingredient, hydroxypropylmethylcellulose or carboxymethylcellulose sodium as binder, hydroxypropylmethylcellulose as a matrix agent, sodium stearyl fumarate as glidant/lubricant.

According to an embodiment of the present invention, the second layer comprises amorphous dapagliflozin as active agent, microcrystalline cellulose and anhydrous lactose as a filler, croscarmellose sodium as disintegrant, colloidal silicon dioxide and sodium stearyl fumarate as glidant/ lubricant.

The present invention is described in more detail by the following examples. The example is not to limit the scope of the invention but should be considered in the light of the details described above.

### Example 1:

| **Active ingredient and excipient** | **Ingredients in one layer (%) (by weight)** |
|---|---|
| **The extended release layer (first layer)** | |
| Metformin HCI | 50.0 - 80.0 |
| Carboxymethylcellulose sodium (7 HF) | 1.0 - 10.0 |
| Hydroxypropylmethylcellulose (K100 LV) | 0.1 -5.0 |
| Hydroxypropylmethylcellulose (K100 MCR/ K100M/ CN1 0T) | 10.0 - 25.0 |
| Sodium stearyl fumarate | 0.01 - 3.0 |
| Pure water | q.s. |
| The extended release layer total | 75.0 - 85.0 |

| The immediate release layer (second layer) | |
|---|---|
| Amorphous dapagliflozin | 0.05 - 5.0 |
| Microcrystalline cellulose | 5.0 - 25.0 |
| Anhydrous lactose | 0.5 - 6.0 |
| Croscarmellose sodium | 0.1 -5.0 |
| Colloidal silicon dioxide | 0.05 - 3.0 |
| Sodium stearyl fumarate | 0.05 - 3.0 |
| **The immediate release layer total** | **10.0 - 25.0** |

| **Film-coating solution** | |
|---|---|
| Moisture-barrier coating | 1.0 - 5.0 |
| **The weight of the film tablet (total)** | **100** |

### Example 2:

| **Active ingredient and excipient** | **Ingredients in one layer (%) (by weight)** |
|---|---|
| **The extended release layer (first layer)** | |
| Metformin HCI | 59.8 |
| Carboxymethylcellulose sodium (7 HF) | 3.0 |
| Hydroxypropylmethylcellulose (K100 LV) | 1.2 |
| Hydroxypropylmethylcellulose (K100 MCR/ K100M/ CN1 0T) | 16.4 |
| Sodium stearyl fumarate | 0.35 |
| Pure water | q.s. |
| The extended release layer total | 80.83 |

| The immediate release layer (second layer) | |
|---|---|
| Amorphous dapagliflozin | 0.6 |
| Microcrystalline cellulose | 11.73 |
| Anhydrous lactose | 2.87 |
| Croscarmellose sodium | 0.9 |
| Colloidal silicon dioxide | 0.3 |
| Sodium stearyl fumarate | 0.35 |
| **The immediate release layer total** | **16.77** |

| **Film-coating solution** | |
|---|---|
| Moisture-barrier coating | 2.4 |
| **The weight of the film tablet (total)** | **100** |

### Example 3:

| | |
|---|---|
| **Active ingredient and excipient** | **Ingredients in one layer (%) (by weight)** |
| **The extended release layer (first layer)** | |
| Metformin HCI | 59.8 |
| Carboxymethylcellulose sodium (7 HF) | 3.0 |
| Hydroxypropylmethylcellulose (K100 LV) | 1.2 |
| Hydroxypropylmethylcellulose (K100 MCR/ K100M/ CN1 0T) | 16.4 |
| Sodium stearyl fumarate | 0.35 |
| Pure water | q.s. |
| **The extended release layer total** | **80.83** |

| **The immediate release layer (second layer)** | |
|---|---|
| Amorphous dapagliflozin | 0.3 |
| Microcrystalline cellulose | 12.0 |
| Anhydrous lactose | 2.87 |
| Croscarmellose sodium | 0.9 |
| Colloidal silicon dioxide | 0.3 |
| Sodium stearyl fumarate | 0.35 |
| **The immediate release layer total** | 16.77 |

| Film-coating solution | |
|---|---|
| Moisture-barrier coating | 2.4 |
| **The weight of the film tablet (total)** | **100** |

The above-mentioned pharmaceutical bilayer tablet at example 1 or 2 or 3 is prepared as follows:
**The first layer (extended release layer):**
   a) Sieving metformin and grinding,
   b) Mixing metformin, carboxymethylcellulose sodium (7 HF), hydroxypropylmethylcellulose (K100 LV) in the granulator and obtained a dry mixture,
   c) Spraying water to the dry mixture and then obtained granules,
   d) Sieving the granules and then drying at fluid bed dryer,
   e) Mixing the granules and hydroxypropylmethylcellulose (K100 MCR/ K100M/ CN10T),
   f) Adding sodium stearyl fumarate and then mixing.
**The second layer (immediate release layer):**
   g) Mixing amorphous dapagliflozin and colloidal silicon dioxide,
   h) Adding anhydrous lactose, croscarmellose sodium and mixing, and then sieving into 630 µ,
   i) Adding the half of microcrystalline cellulose and mixing, then adding the remaining part of microcrystalline cellulose and then mixing and then sieving into 630 µ,
   j) Adding sodium stearyl fumarate and mixing,
**Tablet compression:** The first layer and second layer are compressed to form a bilayer tablet.
**Film coating:** Film coating with moisture-barrier coating.

With the invention, surprisingly, a bilayer tablet formulation of metformin HCI and amorphous dapagliflozin with the desired release profile is obtained. The immediate release and extended release layers in the bilayer tablet have been used in such proportions, so that a formulation with suitable and desired properties could be obtained which provides up to 24 hours of release. The start of treatment is achieved at the desired level depending on the dissolution rate of the immediate release layer.

## Claims

1. A pharmaceutical bilayer tablet comprising;
a. first layer comprising metformin in the form of the free base or in the form of pharmaceutically acceptable salts, crystalline polymorph thereof and at least one pharmaceutically acceptable excipient,
b. second layer comprising amorphous dapagliflozin, and at least one pharmaceutically acceptable excipient,
wherein the composition is free of magnesium stearate.

2. The pharmaceutical bilayer tablet according to claim 1, wherein at least one pharmaceutically acceptable excipient is selected from fillers, binders, disintegrants, matrix agents, glidants/lubricants or mixtures thereof.

3. The pharmaceutical bilayer tablet according to claim 2, wherein fillers are selected from the group comprising microcrystalline cellulose, anhydrous lactose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

4. The pharmaceutical bilayer tablet according to claim 3, wherein the amount of fillers is 5.0% to 30.0% by weight.

5. The pharmaceutical bilayer tablet according to claim 2, wherein binders are selected from the group comprising hydroxypropyl methylcellulose, carboxymethylcellulose sodium, polyvinylpyrrolidone, lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

6. The pharmaceutical bilayer tablet according to claim 5, wherein the binder is hydroxypropyl methylcellulose or carboxymethylcellulose sodium.

7. The pharmaceutical bilayer tablet according to claim 2, wherein disintegrants are selected from the group comprising croscarmellose sodium, crospovidone, sodium starch glycollate, sodium alginate, gums, starch and magnesium aluminum silicate or a mixture thereof.

8. The pharmaceutical bilayer tablet according to claim 7 or 1, wherein the weight ratio of disintegrant to amorphous dapagliflozin is 1.0 - 4.0, preferably 1.2 - 3.5 in the immediate release layer.

9. The pharmaceutical bilayer tablet according to claim 7, wherein the disintegrant is croscarmellose sodium.

10. The pharmaceutical bilayer tablet according to claim 2, wherein glidants/lubricants are selected from group comprising colloidal silicon dioxide, sodium stearyl fumarate, magnesium oxide, starch, silica colloidal anhydrous, talc, polyethylene glycol, stearic acid, aluminum silicate, magnesium silicate, colloidal silica or mixtures thereof.

11. The pharmaceutical bilayer tablet according to claim 10, wherein the glidants/lubricants is colloidal silicon dioxide or sodium stearyl fumarate or mixtures thereof.

12. The pharmaceutical bilayer tablet according to claim 2, wherein matrix agents are selected from the group comprising hydroxypropylmethyl cellulose, ethyl cellulose, polymethylmethacrylate derivatives's, poloxamer, polyvinyl alcohol, xanthan gum, sodium alginate, polymethacrylates, polyacrylamide, hydroxypropyl cellulose, polyvinyl acetate, cellulose acetate phthalate, ethylene vinyl acetate, methyl aminoethyl methacrylate, neutral methacrylic acid esters, polylactide, polylactide co-glycolide, diethyl aminoethyl methacrylate or mixtures thereof.

13. The pharmaceutical bilayer tablet according to claim 12, wherein the matrix agent is hydroxypropyl methyl cellulose.

14. The pharmaceutical bilayer tablet according to claim 1, wherein the first layer comprising metformin in the form of the free base or in the form of pharmaceutically acceptable salts, crystalline polymorph thereof as active ingredient, hydroxypropylmethylcellulose or carboxymethylcellulose sodium as binder, hydroxypropylmethylcellulose as a matrix agent, sodium stearyl fumarate as glidant/lubricant.

15. The pharmaceutical bilayer tablet according to claim 1, wherein the second layer comprising amorphous dapagliflozin as active agent, microcrystalline cellulose and anhydrous lactose as a filler, croscarmellose sodium as disintegrant, colloidal silicon dioxide and sodium stearyl fumarate as glidant/ lubricant.
